**Europäisches Patentamt**

**European Patent Office**

**Office européen des brevets**

(19)

(11) Veröffentlichungsnummer: **0 121 778**
**B1**

(12) # EUROPÄISCHE PATENTSCHRIFT

(45) Veröffentlichungstag der Patentschrift:
**05.08.87**

(21) Anmeldenummer: **84102462.3**

(22) Anmeldetag: **08.03.84**

(51) Int. Cl.⁴: **C 07 H 1/08, C 07 H 21/02,
C 12 P 19/34, B 01 D 13/01**

(54) **Verfahren zur Abtrennung von Ribonukleinsäuren aus einer Lösung, die Desoxyribonukleinsäuren enthält.**

(30) Priorität: **12.03.83 DE 3308932**

(43) Veröffentlichungstag der Anmeldung:
**17.10.84 Patentblatt 84/42**

(45) Bekanntmachung des Hinweises auf die Patenterteilung:
**05.08.87 Patentblatt 87/32**

(84) Benannte Vertragsstaaten:
**AT BE CH DE FR GB IT LI NL SE**

(56) Entgegenhaltungen:
**GB - A - 2 027 033**

(73) Patentinhaber: **HOECHST AKTIENGESELLSCHAFT,
Postfach 80 03 20, D-6230 Frankfurt am Main 80 (DE)**

(72) Erfinder: **Keller, Reinhold, Dr., Wiesenweg 5, D-6232 Bad
Soden am Taunus (DE)**
Erfinder: **Schlingmann, Merten, Dr., Schneidhainer
Strasse 32a, D-6240 Königstein/Taunus (DE)**

# Beschreibung

Die Erfindung betrifft ein Verfahren zur Abtrennung von Ribonukleinsäuren aus einer Lösung, die Desoxyribonukleinsäuren enthält.

Bei der Gewinnung von Protein für die menschliche Ernährung aus mirkobiellen Zellmassen müssen die Nukleinsäuren weitgehend entfernt werden. Vorteilhaft geschieht das nach dem in der Deutschen Patentschrift 26 33 666 (US-Patentschrift 4 206 243) beschriebenen Verfahren, bei dem zunächst die Lipide mit einer Extraktionsmischung aus einem polaren Lösemittel, vorzugsweise Methanol und Ammoniak entfernt werden, worauf die Nukleinsäuren mit Wasser extrahiert werden. In diesen Rohnukleinsäureextrakten liegen neben Ribonukleinsäuren, im folgenden RNA, auch Desoxyribonukleinsäuren, im folgenden DNA, vor.

5'-Ribonukleotide dienen als Ausgangsstoffe zur Herstellung von Lebensmittelzusatzstoffen und für Arzneimittel. Bekannt ist ihre Herstellung durch enzymatische Hydrolyse von RNA. Das hierzu verwendete Enzym 5'-Phosphodiesterase hydrolysiert jedoch neben RNA gleichzeitig auch DNA, so dass neben den gewünschten 5'-Ribonukleotiden als Nebenprodukte auch 5'-Desoxyribonuleotide entstehen. Diese Nebenprodukte sind von den 5'-Ribonukleotiden nur sehr schwierig abzutrennen. Es sind deshalb schon Verfahren zur Herstellung von reiner RNA bekannt geworden. Hierzu gehört die selektive Ausfällung von RNA durch Erhitzen und anschliessende Säurebehandlung, wie es in der japanischen Patentanmeldung 78-20 493 beschrieben ist. Nach dem Verfahren der japanischen Patentanmeldung 79-55 791 erfolgt die Säurefällung von RNA in Gegenwart zweiwertiger Kationen. Bei diesen Verfahren wird also die DNA durch eine Hitze- und Säurebehandlung zersetzt. Weiterhin geht hierbei auch ein beträchtlicher Anteil der RNA verloren.

Es wurde schon ein Verfahren zur Herstellung von 5'-Ribonukleotiden vorgeschlagen, das dadurch gekennzeichnet ist, dass man eine RNA enthaltende Lösung von Rohnukleinsäuren mit einer an einem polymeren Träger immobilisierten 5'-Phosphodiesterase selektiv hydrolysiert und aus dem Hydrolysat die unveränderte DNA und die 5'-Ribonukleotide durch bekannte Reinigungs- und Trennverfahren isoliert (Deutsche Offenlegungsschrift 31 36 940).

Es wurde nun gefunden, dass man aus Rohnukleinsäurelösungen die RNA von der DNA abtrennen kann, wenn man diese Lösung einem Membrantrennverfahren unterwirft. Man macht hierbei von dem Molgewichtsunterschied zwischen der relativ hochmolekularen DNA und der relativ niedermolekularen RNA Gebrauch. Die Ausschlussgrenze der Membran wählt man nach den bekannten bzw. ermittelten Molgewichten der zu trennenden Nukleinsäuren.

Membrantrennprozesse sind allgemein und insbesondere in der Biotechnologie geläufig (Übersichtsartikel: H. Strathmann, Chemie-Technik 11 [1982] 813–819). Bevorzugt für das erfindungsgemässe Verfahren ist die Ultrafiltration in Platten-, Rohr-, Kapillarrohr-, Wickelmembran- und insbesondere Hohlfaserapparaten.

Als Ausgangsmaterialien dienen bevorzugt Rohnukleinsäuren aus Bakterien, in denen die RNA zur DNA üblicherweise in einem Verhältnis von etwa 4:1 vorliegt. Wichtig ist, dass Rohnukleinsäurelösungen eingesetzt werden, bei denen die DNA nicht oder nur unwesentlich abgebaut wurde, also Ausgangslösungen, in denen der natürliche Molgewichtsunterschied zwischen DNA und RNA im wesentlichen erhalten blieb. Auch in dieser Beziehung ist das aus der Deutschen Patentschrift 26 33 666 bekannte Verfahren vorteilhaft.

Die erfindungsgemäss erhaltenen Permeate können in bekannter Weise, beispielsweise enzymatisch, zu den 5'-Ribonukleotiden abgebaut werden. Die Retentate können in gleicher Weise zu den 5'-Desoxyribonukleotiden weiterverarbeitet werden, die in vielfältiger Weise Verwendung finden, beispielsweise in der Gentechnologie. In den folgenden Beispielen wird die Erfindung näher erläutert.

Beispiel 1

Eine gemäss der Deutschen Patentschrift 26 33 451 (US-Patentschrift 4 166 004), Beispiel 2, erhaltene Bakterienmasse mit einem Nukleinsäuregehalt von 11,2 Gew.-% und einer Restfeuchte von 2 bis 4 Gew.-% wurde 30 Minuten in einem Wirbelbett bei 160 °C Lufttemperatur behandelt, wobei 10 Minuten eine Produkttemperatur von 120 °C eingehalten wurde. Diese thermisch nachbehandelte Zellmasse wurde dann gemäss Beispiel 1 der Deutschen Patentschrift 26 33 666 mit methanolischem Ammoniak extrahiert, mit Methanol gewaschen und 5 Stunden im Vakuum bei 40 °C getrocknet.

Die trockene Zellmasse wurde in der 10-fachen Gewichtsmenge Wasser suspendiert und durch Rühren homogenisiert. Nach Erhöhen der Temperatur auf 55 °C wurde noch 20 Minuten weitergerührt, auf 30 °C abgekühlt und durch Zentrifugieren in eine feste und flüssige Phase getrennt. Das erhaltene Sediment wurde erneut mit der gleichen Menge Wasser vermischt und 10 Minuten bei 20 °C gerührt. Danach wurde erneut zentrifugiert und die flüssigen Phasen vereinigt. Sie enthalten 9 g Nukleinsäuren pro Liter bei einem RNA/DNA-Verhältnis von 4:1.

100 l dieser Rohnukleinsäure-Lösung werden mit einem Tiefenfilter klarfiltriert und das Filtrat in ein Hohlfaser-Ultrafiltrationsgerät (Fa. Amicon, DC 50 EM, Hollow-Fiber-Patrone, Mol-Gew.-Trenngrenze von 100 000) geleitet. Die Rohnukleinsäure-Lösung wurde dabei bis auf 10 l aufkonzentriert und anschliessend mit 10 l entionisiertem Wasser diafiltriert.

Das so erhaltene Retentat enthält die DNA mit einer Verunreinigung von weniger als 1% RNA und das Permeat die RNA ohne DNA-Verunreinigung.

2

Zur Isolierung der DNA bzw. RNA wird das Retentat bzw. das Permeat auf 5 °C abgekühlt und der pH-Wert der Lösung mit Salzsäure auf 2,0 gestellt. Die ausgefallene DNA bzw. RNA wird abzentrifugiert und getrocknet.

Beispiel 2

Man geht von einer Rohnukleinsäure-Lösung aus, die gemäss Beispiel 8 der Deutschen Patentschrift 26 33 666 erhalten wurde, bei der jedoch die Nukleinsäuren nur mit Wasser extrahiert wurden. Diese Rohnukleinsäure-Lösung wird auf einen Gehalt von 30 g Nukleinsäuren pro Liter aufkonzentriert. Das RNA/DNA-Verhältnis betrug 3:1.

50 l dieser Rohnukleinsäure-Lösung wurden wie im Beispiel 1 beschrieben vorfiltriert und anschliessend ultrafiltriert. Das bis auf 5 l aufkonzentrierte Retentat wurde mit 5 l entionisiertem Wasser dialysiert. Aus dem so gewaschenen Retentat wird die DNA nach Abkühlen auf 5 °C durch Einstellen des pH-Wertes auf 2,0 gefällt und isoliert. Analog wird die RNA aus dem Permeat gewonnen.

**Patentansprüche**

1. Verfahren zur Abtrennung der Desoxyribonukleinsäure von Ribonukleinsäure aus Rohnukleinsäure-Lösungen, dadurch gekennzeichnet, dass man die Rohnukleinsäure-Lösung einem Membran-Trennverfahren unterwirft, wobei die Desoxyribonukleinsäure zurückgehalten wird und die Ribonukleinsäure permeiert.

2. Verfahren nach Anspruch 1, dadurch gekennzeichnet, dass die Trennung als Ultrafiltration mit Hohlfasermembranen erfolgt.

3. Verfahren nach Anspruch 1 oder 2, dadurch gekennzeichnet, dass eine Membran mit einer Mol-Gew.-Trenngrenze von 100 000 eingesetzt wird.

**Revendications**

1. Procédé de séparation de l'acide désoxyribonucléique d'avec l'acide ribonucléique à partir de solutions d'acides nucléiques bruts, caractérisé en ce que l'on soumet la solution d'acide nucléique brut à un procédé de séparation par membrane, l'acide désoxyribonucléique étant retenu et l'acide ribonucléique passant par perméation.

2. Procédé selon la revendication 1, caractérisé en ce que la séparation est une ultrafiltration avec des membranes à fibres creuses.

3. Procédé selon la revendication 1 ou 2, caractérisé en ce que l'on emploie une membrane ayant une limite de séparation par poids moléculaire de 100 000.

**Claims**

1. A process for separating the deoxyribonucleic acid from ribonucleic acid in crude nucleic acid solutions comprises subjecting the crude nucleic acid solution to a membrane separating process, the deoxyribonucleic acid being held back and the ribonucleic acid permeating through.

2. The process as claimed in claim 1, wherein the separation is carried out as ultrafiltration with hollow fiber membranes.

3. The process as claimed in claim 1 or 2, wherein a membrane with a molecular weight separating limit of 100 000 is employed.